# EUROPÄISCHE PATENTSCHRIFT

(11) **EP 2 852 970 B1**
(45) Veröffentlichungstag und Bekanntmachung des Hinweises auf die Patenterteilung: **06.01.2021**
(21) Anmeldenummer: 12722392.3
(22) Anmeldetag: 22.05.2012
(51) Int. Cl.: H01L 21/60, H01L 21/56, H01L 23/48, H01L 23/367, A61M 5/142, A61N 1/36, A61N 1/362

(54) **VERFAHREN ZUM HERSTELLEN EINER ELEKTRONISCHEN BAUGRUPPE**
METHOD FOR PRODUCING AN ELECTRONIC SUBASSEMBLY
PROCÉDÉ DE FABRICATION D'UN MODULE ÉLECTRONIQUE

(43) Veröffentlichungstag der Anmeldung: 01.04.2015
(73) Patentinhaber: Würth Elektronik GmbH & Co. KG, 74676 Niedernhall (DE)
(72) Erfinder: KOSTELNIK, Jan, 74592 Kirchberg (DE)
(74) Vertreter: Richardt Patentanwälte PartG mbB
(86) Internationale Anmeldenummer: PCT/EP2012/059476
(87) Internationale Veröffentlichungsnummer: WO 2013/174418

(56) Entgegenhaltungen:
- DE-A1-102008 040 488
- DE-A1-102010 014 579
- DE-A1-102011 000 530
- JP-A- 2008 141 007
- US-A1- 2011 233 754

## Beschreibung

Die Erfindung betrifft ein Verfahren zum Herstellen einer elektronischen Baugruppe.

Ein derartiges Verfahren ist bekannt aus DE 10 2008 009 220 A1 und aus DE 10 2010 014 579 A1.

Die Druckschrift DE 10 2011 000 530 A1 offenbart ein Verfahren zur Herstellung einer Halbleiteranordnung. In einem Ausführungsbeispiel wird auf eine Metallfolie eine Paste aufgebracht und in diese Paste ein Halbleiterchip eingedrückt.

Die Druckschrift JP2008141007 offenbart ein Verfahren zur Herstellung eines MultiLayer-Substrats. Dabei wird ein elektronisches Bauteil zwischen mehreren Harz-Filmen angeordnet, um diesen vor Beschädigungen zu schützen und elektrisch zu kontaktieren.

Die Druckschrift DE 10 2008 040 488 A1 offenbart eine elektronische Baueinheit und ein Verfahren zu deren Herstellung.

Aufgabe der vorliegenden Erfindung ist es demgegenüber, ein verbessertes Verfahren und eine verbesserte elektronische Baugruppe anzugeben.

Diese Aufgabe wird verfahrensseitig gelöst durch
1.1 Bereitstellen einer elektrisch leitenden Folie, insbesondere einer Trägerfolie,
1.2 Bereitstellen mindestens eines elektrischen Bauteils mit mindestens einer elektrischen Kontaktstelle,
1.3 Aufbringung eines Klebers zwischen dem elektrischen Bauteil und einer Oberfläche der elektrisch leitenden Folie,
1.4 Anordnen des mindestens einen Bauteils mit der mindestens einen elektrischen Kontaktstelle auf der Oberfläche der elektrisch leitenden Folie, und Fixierung des mindestens einen Bauteils durch Ausbildung einer Klebeverbindung zwischen dem elektrischen Bauteil und der Oberfläche,
1.5 Bereitstellen eines Trägers, insbesondere aus einem flexiblen Material,
1.6 Laminieren der Folie mit dem Träger derart, dass das mindestens eine elektrische Bauteil zwischen Folie und Träger angeordnet ist, und Bilden einer mechanischen und elektrischen Verbindung zwischen der elektrischen Kontaktstelle des mindestens einen elektrischen Bauteils und der elektrisch leitenden Folie durch Niedertemperatursintern von Nanopartikeln, insbesondere aus Gold, Silber, Nickel oder Kupfer oder aus einer Legierung aus diesen Metallen, wobei das Laminieren der Folie gleichzeitig mit dem Niedertemperatursintern erfolgt,
1.7 Strukturieren der elektrisch leitenden Folie zu Leiterbahnen undloder Kühlflächen.

Ausführungsform der Erfindung sind besonders vorteilhaft, da das Laminieren und das Niedertemperatursintern in demselben Prozessschritt im Wesentlichen gleichzeitig erfolgen können. Dies wird einerseits durch die Verwendung von Nanopartikeln ermöglicht, welche ein Niedertemperatursintern in einem Temperaturbereich ermöglichen, der auch für das Laminieren der Folie geeignet ist, und andererseits durch die vorherige Fixierung des mindestens einen elektrischen Bauteils auf der Oberfläche der elektrisch leitenden Folie. Durch diese Fixierung ist sichergestellt, dass sich das elektrische Bauteil an der richtigen Stelle befindet, wenn der Träger für das nachfolgende Laminieren auf die Folie und die im Raum befindlichen Bauteile aufgebracht wird, und dass das Bauteil nicht verrutschen kann.

Unter einem "elektrischen Bauteil" werden hier insbesondere gehauste und ungehauste elektronische Bauelemente, wie zum Beispiel integrierte Halbleiterchips, zum Beispiel sogenannte Nacktchips ("bare chip" oder "bare die") verstanden sowie auch diskrete elektrische oder elektronische Bauelemente, wie zum Beispiel Kondensatoren.

Ein solches elektrisches Bauteil hat üblicherweise eine Seite, auf der eine oder mehrere elektrische Kontaktstellen zur elektrischen Kontaktierung des Bauteils vorgesehen sind. Diese Seite des Bauteils wird auch als "aktive Seite" des Bauteils bezeichnet. Insbesondere wenn es sich bei dem Bauteil um ein bare die handelt, sind die elektrischen Kontaktstellen als sogenannte contact pads ausgebildet.

Bei dem Träger kann es sich um vorimprägnierte Fasern (preimpregnated fibers, das heißt ein sogenanntes Prepreg) handeln, worunter hier eine ungehärtete duroplastische Kunststoffmatrix verstanden wird. Beispielsweise kann es sich bei dem Träger um ein flüssigkristallines Polymer (liquid crystal polymere - LCP) handeln, was besonders vorteilhaft zur Herstellung biokompatibler Implantate ist, oder ein anderes Trägermaterial.

Nach einer Ausführungsform der Erfindung wird als Ausgangsmaterial eine elektrisch leitende Folie verwendet, deren Oberfläche mit Nanopartikeln beschichtet ist. Das mindestens eine elektrische Bauteil wird dann auf der Oberfläche der elektrisch leitenden Folie durch einen Kleber fixiert, der außerhalb der elektrischen Kontaktstellen des Bauteils aufgebracht wird. Vorzugsweise handelt es sich bei dem Kleber um einen nicht leitfähigen Kleber, das heißt ein sogenanntes non-conducting-adhesive (NCA) oder eine non-conducting paste (NCP). Wenn es sich beispielsweise bei dem elektrischen Bauteil um ein bare die handelt, welches außerhalb der elektrischen Kontaktstellen von einer Passivierungsschicht umgeben ist, so wird die Klebeverbindung mithilfe dieses Klebers zwischen der Passivierungsschicht und der Oberfläche der elektrisch leitenden Folie hergestellt.

Nach einer Ausführungsform der Erfindung wird als Ausgangsmaterial eine elektrisch leitende Folie verwendet, deren Oberfläche nicht mit Nanopartikeln beschichtet sein muss. In diesem Fall wird ein Kleber verwendet, der die Nanopartikel beinhaltet. Der Kleber wird auf die mindestens eine elektrische Kontaktstelle des elektrischen Bauteils aufgebracht, um das elektrische Bauteil auf der Oberfläche der elektrisch leitenden Folie zu fixieren. Anschließend wird der Träger aufgebracht, um die Folie mit dem Träger zu laminieren und gleichzeitig die Niedertemperatursinterung durchzuführen. Hierbei versintern die in der Klebeverbindung beinhalteten Nanopartikel. Diese Ausführungsform des erfindungsgemäßen Verfahrens ist besonders vorteilhaft, da durch die Aufbringung des Klebers auch gleichzeitig die Nanopartikel aufgebracht werden, die für das anschließende Niedertemperatursintern erforderlich sind.

Nach einer Ausführungsform der Erfindung wird das Niedertemperatursintern als "druckloses" Niedertemperatursintern durchgeführt. Unter "drucklos" wird hierbei verstanden, dass das Niedertemperatursintern nicht bei den üblicherweise anzuwendenden hohen Drücken von zum Beispiel 200 bar, d.h. 2000 kPa, durchgeführt wird, sondern bei einem wesentlich geringeren Druck, wie er auch für das Laminieren verwendet wird, das heißt bei einem Druck von beispielsweise 15 - 20 bar. , d.h. 1500-2000 kPa.

Entsprechende Kleber oder Pasten, die Nanopartikel für eine solche drucklose Niedertemperatursinterung enthalten sind an sich aus dem Stand der Technik bekannt, wie z.B. aus DE 10 2008 039 828 A1, und sind kommerziell von der Firma Heraeus unter dem Handelsnamen mAgic Adhesive bzw. mAgic Paste erhältlich.

Hierdurch wird ein besonders effizientes und Material sparendes Herstellungsverfahren ermöglicht, da die Menge der eingesetzten Nanopartikel minimiert werden kann. Außerdem ist die so hergestellte Verbindung besonders mechanisch stabil und hat gut elektrische Kontakteigenschaften aufgrund der kovalenten Bindungen der Nanopartikel, die zu einem geringen Übergangswiderstand führen.

Nach einer Ausführungsform der Erfindung ist die Größe der Nanopartikel so gewählt, dass diese bei Raumtemperatur chemisch relativ inert sind, sodass bei Raumtemperatur keine spontane Versinterung erfolgt. Andererseits ist die Größe der Nanopartikel hinreichend klein gewählt, sodass in dem für das Laminieren erforderlichen Temperaturbereich die Reaktionsfähigkeit der Nanopartikel im Vergleich zu makroskopischen Partikeln bereits so weit erhöht ist, dass es zur Ausbildung kovalenter chemischer Verbindungen zwischen den Nanopartikeln kommt, sodass diese also miteinander versintern.

Nach einer Ausführungsform der Erfindung kann es sich bei dem Kleber um ein LCP handeln. Dies ist besonders vorteilhaft, wenn auch der Träger durch ein LCP gebildet wird, insbesondere im Hinblick auf die thermische Belastbarkeit und die Langzeitstabilität der resultierenden elektronischen Baugruppe, was besonders für Anwendungen im medizinischen Bereich, insbesondere für Implantate, vorteilhaft ist.

In einem weiteren Aspekt betrifft die Erfindung ein Verfahren zum Herstellen einer elektronischen Baugruppe, bei dem vor dem Laminieren der Folie mit dem Träger das auf der elektrisch leitenden Folie angeordnete mindestens eine Bauteil mit einem Füllmaterial aus einem Polymer umhüllt wird, wobei bereits bei diesem Schritt des Umhüllens das Niedertemperatursintern erfolgt. Dies ist besonders vorteilhaft, wenn das resultierende Zwischenprodukt in einem diskontinuierlichen Prozess von einer Fertigungsstation zu einer anderen transportiert werden muss sowie auch für die mechanische Stabilität der resultierenden elektronischen Baugruppe.

In einem weiteren Aspekt betrifft die Erfindung eine elektronische Baugruppe, die nach einem erfindungsgemäßen Verfahren hergestellt worden ist. Insbesondere kann es sich hierbei um ein implantat handeln, wie zum Beispiel ein Elektronikmodul für eine Insulinpumpe, einen Herzschrittmacher oder ein implantierbares Hörgerät.

Vorteilhaft erfolgt das Bereitstellen der Folie durch das Bereitstellen einzelner, zugeschnittener Folienabschnitte oder durch das Bereitstellen einer Endlosfolie von einer Rolle.

Bevorzugt erfolgt das Bereitstellen des mindestens einen elektrischen Bauteils durch Bereitstellen desselben auf einer Endlosfolie oder Bereitstellen in einem Magazin.

Vorteilhaft erfolgt das Anordnen des mindestens einen elektrischen Bauteils automatisiert durch einen Bestückungsroboter oder von Hand, insbesondere mit Hilfe von Schablonen oder Positionsmarkern in bzw. auf der Folie.

Das Bereitstellen des Trägers kann durch Bereitstellen einzelner, zugeschnittener Trägerabschnitte oder durch Bereitstellen eines Endlosträgers von einer Rolle erfolgen.

Vorzugsweise erfolgt das Laminieren der Folie mit dem Träger durch Ausüben eines Anpressdruckes auf die Folie in Richtung des Trägers, insbesondere unter gleichzeitiger Wärmeeinwirkung.

Vorteilhaft erfolgt das Strukturieren der elektrisch leitenden Folie zu Leiterbahnen und ggfs. Kühlflächen durch Direktstrukturierung, z. B. mit einem Laser, oder durch Erzeugen einer Positiv- oder Negativmaske und anschließendem Ätzen.

Vorteilhaft wird die Folie mit dem Träger und dem mindestens einen elektrischen Bauteil nach der Strukturierung der Folie auf einer Rolle aufgewickelt.

In Weiterbildung der Erfindung kann vorgesehen sein, dass ein Umhüllen des mindestens einen elektrischen Bauteils nach der Anordnung des Bauteils auf der Folie mit einem Füllmaterial aus einem Polymer, insbesondere einem Thermoplast, Duroplast oder einem Elastomer erfolgt.

Vorteilhaft erfolgt das Füllen des Füllmaterials in eine vorbestimmte Form, insbesondere durch Giessen, Schäumen, Extrudieren oder Laminieren.

In Weiterbildung der Erfindung kann vorgesehen sein, dass ein Erzeugen mindestens einer weiteren Trägerschicht auf den durch Strukturierung erzeugten Leiterbahnen erfolgt, wobei diese bevorzugt isolierend und/oder dieelektrisch ist und/oder weitere Leiterbahnen enthält.

Insbesondere kann nach dem Schritt 1.6 ein Aufbringen mindestens einer Verstärkungsschicht, vorzugsweise mehrerer Verstärkungsschichten, auf den Träger oder die Trägerschicht erfolgen. Es kann alternativ auch vorgesehen sein, dass der Träger ggfs. nach dem Schritt 1.6 entfernt wird.

In Weiterbildung der Erfindung kann ein Zuschneiden des Trägers in eine vorbestimmte Form zum Bilden der elektronischen Baugruppe, insbesondere vor und/oder nach dem Laminieren mit der Leiterplatte im Schritt 1.6. erfolgen.

Dabei kann die elektrisch leitende Folie aus Metall, insbesondere Kupfer, elektrisch leitfähigem Kunststoff oder elektrisch leitfähiger Keramik bestehen, auf deren Oberfläche ggfs. zumindest bereichsweise eine Schicht aus Silber oder einer Silberverbindung und/oder eine dreidimensionale Struktur aus metallischen Nanopartikeln vorhanden ist und ggfs. durch eine Trägerfolie verstärkt ist.

Insbesondere kann vorgesehen sein, dass die elektrisch leitende Folie auf der dem mindestens einem elektrischen Bauteil zugewandten Seite glatt oder angerauht ist oder eine Höckerstruktur aufweist.

Es kann vorgesehen sein, dass das mindestens eine elektrische Bauteil ein passives oder aktives Bauelement, insbesondere ein Chip, ist, wobei es vorzugsweise außerhalb der Kontaktstellen mit einem Schutzüberzug, insbesondere einem Lack, d.h. einer sog. Passivierungsschicht versehen ist.

In Weiterbildung kann vorgesehen sein, dass der Schutzüberzug elektrisch isolierend und/oder ein Dieelektrikum ist.

Vorteilhaft kann der Träger eine Folie, insbesondere aus Metall, Kunststoff oder Keramik sein, eine Matte aus Naturmaterialien, wie z. B. Pflanzenfasern, ein Lederabschnitt, ein Textilabschnitt oder ein Abschnitt aus einem sonstigen Gewebe oder Verbundwerkstoff sein oder eine Leiterplatte, ein Keramikträger oder ein Glasträger sein.

In Weiterbildung der Erfindung kann vorgesehen sein, dass dem Träger eine Verstärkungsschicht zugeordnet ist und insbesondere aus Metall, Kunststoff oder Keramik sein kann, aus Naturmaterialien, wie z. B. Pflanzenfasern, aus Leder, aus Textil oder aus einem sonstigen Gewebe oder Verbundwerkstoff sein kann oder eine Leiterplatte, ein Keramikträger oder ein Glasträger sein kann.

Das mindestens eine elektrische Bauteil kann mindestens eine Kontaktseite aufweisen, in der jeweils mindestens eine elektrische Kontaktstelle angeordnet ist, wobei vorzugsweise die mindestens eine elektrische Kontaktstelle plan mit der entsprechenden Kontaktseite ist.

Vorteilhaft besteht die Kontaktstelle aus Kupfer oder einer Kupferverbindung, auf deren Oberfläche ggfs. eine Schicht aus Silber oder einer Silberverbindung und/oder eine dreidimensionale Struktur aus metallischen Nanopartikeln vorhanden sein kann.

In Weiterbildung kann vorgesehen sein, dass an dem mindestens einem elektrischen Bauteil ein Abschnitt der elektrisch leitenden Folie als Wärme abführender Kontaktbereich gebildet ist (Folienkühlabschnitt).

In Weiterbildung der Erfindung kann vorgesehen sein, dass ein Kühlkörper oder ein Kühlkanal mit dem Folienkühlabschnitt und/oder dem mindestens einem elektronischen Bauteil verbunden ist.

Weitere Einzelheiten, Merkmale und Vorzüge der Erfindung ergeben sich aus den Patentansprüchen, deren Wortlaut durch Bezugnahme zum Inhalt der Beschreibung gemacht wird, der folgenden Beschreibung bevorzugter Ausführungsformen der Erfindung sowie anhand der Zeichnungen. Hierbei zeigen:
fig. 1 schematisch die Seitenansicht einer elektrisch leitenden Folie als Ausgangsmaterial für das Verfahren;
fig. 2 schematisch einen Querschnitt eines elektrischen Bauteils;
fig. 2a einen vergrößerten Ausschnitt der fig. 2;
fig. 2b eine vergrößerte Darstellung einer Kontaktstelle des Bauteils der fig. 2a;
fig. 3 eine Seitenansicht der Folie der fig. 1 mit zwei darauf angeordneten Bauteilen;
fig. 4 die Anordnung der fig. 3, wobei zusätzlich ein Füllmaterial aufgebracht ist;
fig. 5 die Anordnung der fig. 4, wobei zusätzlich ein Träger auflaminiert ist;
fig. 6 die Anordnung der fig. 5, wobei zusätzlich Leiterbahnen und ein Folienkühlabschnitt angeordnet sind;
fig. 7 die Anordnung der fig. 6 , wobei zusätzlich Leiterbahnen und ein Folienkühlabschnitt angeordnet sind, wobei aber der Träger 9 wieder entfernt und eine Trägerschicht 9a mit einer weiteren Verstärkungsschicht 9b auf der Seite der Leiterbahnen auflaminiert wurde; und
fig. 8 schematische Ansicht einer elektronischen Baugruppe
fig. 9 eine perspektivische Darstellung von Klebeverbindungen,
fig. 10 schematisch die Seitenansicht einer leitenden Folie als Ausgangsmaterial für eine weitere Ausführungsform eines erfindungsgemäßen Verfahrens;
fig. 11 den gleichen Abschnitt der Folie mit aufgebrachten Kleberpunkten;
fig. 12 die gleiche Ansicht mit zwei mithilfe der Kleberpunkte verklebten elektronischen Bauteilen;
fig. 13 den Zustand der Herstellung nach dem Aufbringen einer Umhüllung;
fig. 14 den Zustand nach dem Zuschneiden einer elektronischen Baugruppe;
fig. 15 den Endzustand einer nach dem Verfahren hergestellten Mehrlagenleiterplatte;
fig. 16 die schematische Seitenansicht eines Ausgangsmaterials nach einer weiteren Ausführungsform des erfindungsgemässen Verfahrens;
fig. 17 den Zustand nach einem weiteren Verfahrensschritt;
fig. 18 den Zustand nach einem weiteren Verfahrensschritt;
fig. 19 einen weiteren Verfahrensschritt;
fig. 20 das Abziehen der Trägerfolie;
fig. 21 eine der fig. 20 entsprechende Darstellung bei einer Abwandlung;
fig. 22 eine Ausgangssituation bei einer nochmals weiteren Ausführungsform;
fig. 23 den der fig. 21 entsprechenden Zustand der Ausführungsform nach fig. 22;

Im Weiteren werden einander entsprechende oder identische Elemente der verschiedenen Ausführungsformen jeweils mit denselben Bezugszeichen gekennzeichnet.

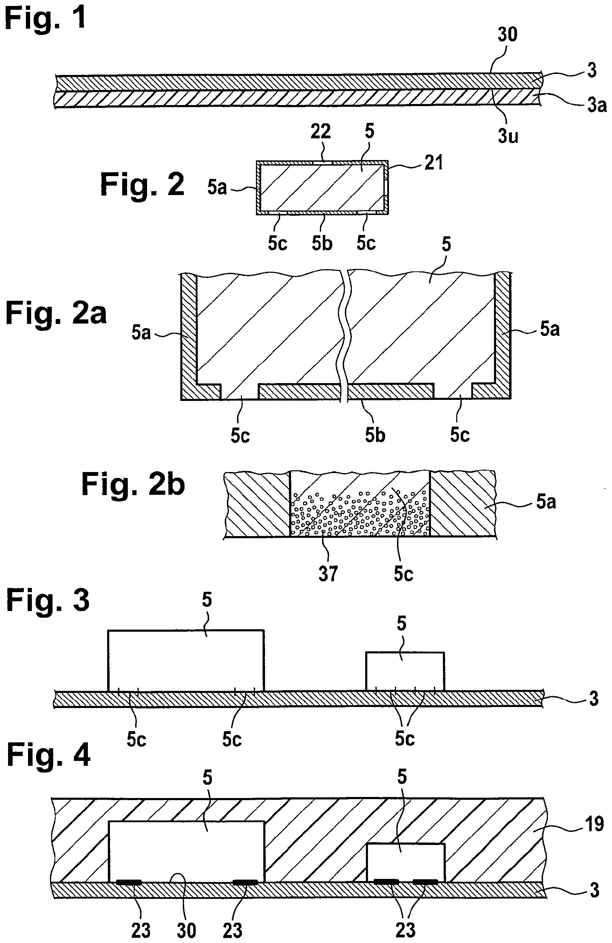

fig. 1 zeigt schematisch einen Ausschnitt einer Seitenansicht einer von einer Rolle abgewickelten, elektrisch leitenden Folie 3 aus Kupfer. Eine Folienoberfläche 3o , welche nachher einem elektrischen Bauteil 5 zugewandt ist, ist mit einer gleichmäßigen Beschichtung von Nanopartikeln, beispielsweise Silberpartikeln einer Partikelgröße von ca. 20-300 nm oder einer silberhaltigen Legierung versehen. Die Folie 3 ist an der der Seite 3o gegenüberliegenden Seite durch eine Trägerfolie 3a verstärkt, die auf die Folie 3 auflaminiert ist und aus einem Polymer besteht.
fig. 2 zeigt schematisch ein elektrisches Bauteil 5 , vorliegend einen Chip, in einer Schnittansicht. Das Bauteil 5 ist an allen Seiten von einer Schutzschicht 5a aus elektrisch isolierendem Lack überzogen, d.h. einer sog. Passivierungsschicht. Das Bauteil 5 weist eine Kontaktseite 5b, die auch als aktive Seite bezeichnet wird, mit zwei Kontaktstellen 5c für die elektrische Kontaktierung mit der Folie 3 auf, die von der aufgetragenen Schutzschicht 5a ausgespart sind. Zusätzlich kann das Bauteil 5 weitere Kontaktstellen haben, wie z.B. an dessen Kante 21 oder an der Oberseite 22. Die Oberflächen der Kontaktstellen 5c sind, wie aus fig. 2b ersichtlich, mit einer Schicht Silber (oder einer silberhaltigen Legierung) beschichtet, nämlich mit Nanopartikeln 37. Die Nanopartikel können teilweise in die Kontaktstellen 5c hinein diffundieren, was einen besonders geringen elektrischen Übergangswiderstand ergibt. Die Bauteile 5 werden von einer von einer Rolle abgewickelten Folie bereitgestellt, auf der sie zueinander beabstandet angeordnet sind. Beispielsweise handelt es sich bei dem Bauteil 5 um ein bare die, wobei die Kontaktstellen 5c dann als sog. contact pads ausgebildet sind.

Ein Roboterarm greift jeweils ein oder mehrere Bauteile 5 von der Folie und setzt diese an definierten Stellen auf der beschichteten Oberfläche 3o der Folie 3 auf. Dabei wird ein Kleber zwischen dem Bauteil 5 und der Oberfläche 30 der elektrisch leitenden Folie aufgebracht, um das Bauteil durch Ausbildung einer Klebeverbindung zu fixieren, wobei es sich bei der Verbindung 7 um eine Klebeverbindung handelt.

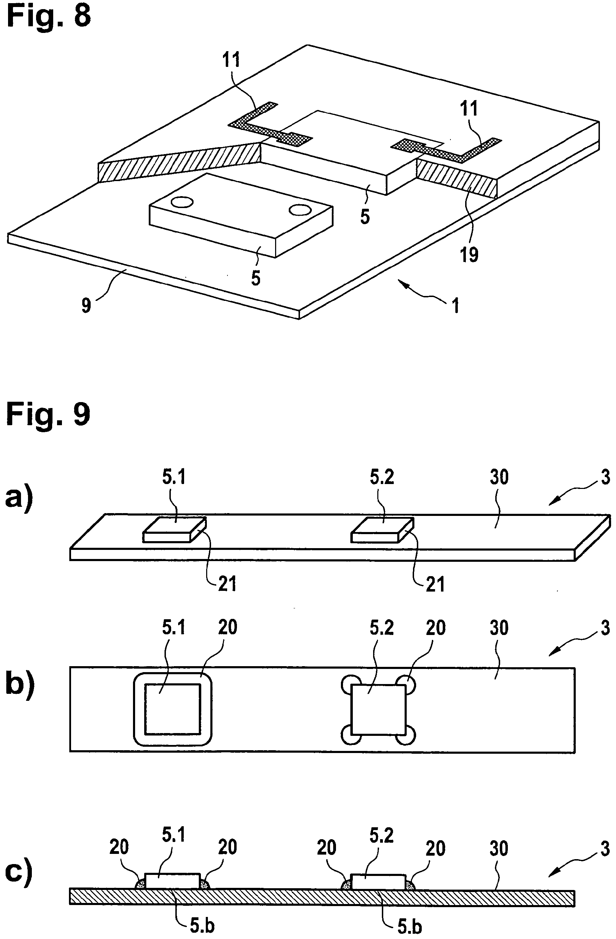

Der Kleber wird vor dem Absetzen eines der Bauteile 5 auf die Oberfläche 30 der Folie aufgebracht. In einem Beispiel, das nicht unter die beanspruchte Erfindung fällt, kann der Kleber, beispielsweise ebenfalls robotisch, außerhalb der elektrischen Kontaktstellen 5c auf die aktive Seite, d.h. die Kontaktseite 5b des Bauteils 5 aufgebracht werden. Ein weiteres Beispiel, das nicht unter die beanspruchte Erfindung fällt ist, dass zunächst das Bauteil von dem Roboterarm auf der Oberfläche 30 der elektrisch leitenden Folie positioniert wird, um das Bauteil dort anzuordnen, und dass anschließend der Kleber zur Herstellung der Klebeverbindung aufgebracht wird, beispielsweise indem der Kleber entlang der seitlichen Kante des Bauteils aufgebracht wird (vgl. hierzu auch die Beispiele gemäß Fig. 9).

Die auf diese Weise hergestellte Klebeverbindung fixiert die Bauteile 5 mechanisch auf der Folie 3 ohne sie elektrisch mit ihr zu kontaktieren. Vorzugsweise wird bei diesem Beispiel der Kleber 20 (vgl. Fig. 9) außerhalb der elektrischen Kontaktstellen 5c aufgebracht, um die anschließende Ausbildung der mechanischen und elektrischen Verbindung 23 (vgl. Fig. 4) durch das Niedertemperatursintern nicht zu behindern, zumal der Kleber 20 bei diesem Beispiel <eine metallischen Nanopartikel enthält.
fig. 3 zeigt die Anordnung von zwei derartig befestigten Bauteilen 5 auf der Folie 3. Nach einer Ausführungsform der Erfindung wird von einer weiteren Rolle eine Folie eines thermoplastischen Polymers abgerollt und auf die Anordnung der fig. 3 aufgelegt. Beides wird durch einen Spalt zwischen zwei erwärmten Walzen geführt, um die Folie des thermoplastischen Polymers zu erwärmen und diese gegen die Folie 3 zu drücken, um die Bauteile 5 zu umhüllen. Dadurch entsteht eine Umhüllung der Bauteile 5 mit einem Füllmaterial 19 (dem thermoplastischen Polymer) auf der Folie 3, die die Bauteile 5 vor Umwelteinflüssen schützt, wie in fig. 4 dargestellt.

Bei diesem Schritt kann es bereits zu der Niedertemperatursinterung, d.h. zur Ausbildung einer mechanischen und elektrischen Verbindung 23 zwischen den Kontaktstellen 5c und der Oberfläche 30 kommen, wenn die Walzen hinreichend warm sind. Von einer weiteren Rolle wird nun eine Folie eines Kunststoffverbundmaterials, vorliegend in einem Epoxyharz angeordnete Glasfasern, abgewickelt und auf die Anordnung der fig. 4 aufgelegt. Beides wird durch einen Spalt zwischen zwei erwärmten Walzen geführt, um die Folie des Kunststoffverbundmaterials zu erwärmen und diese gegen die Umhüllung, d.h. das Füllmaterial 19 zu drücken, um das Kunststoffverbundmaterial auf die Umhüllung aufzulaminieren.

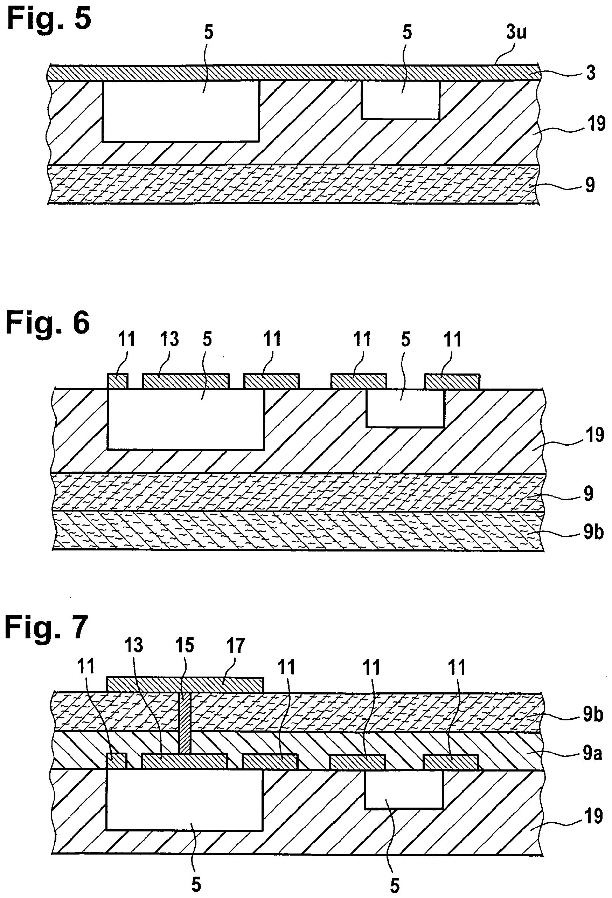

Wie in fig. 5 dargestellt, bildet das auflaminierte Kunststoffverbundmaterial einen Träger 9 für die Anordnung der fig. 4 , vorliegend einen starren Träger 9. Nach der Erfindung wird bei diesem Schritt die Niedertemperatursinterung zur Ausbildung der mechanischen und elektrischen Verbindung 23 zwischen den Kontaktstellen 5c und der Oberfläche 30 durchgeführt, indem die Laminierung in einem dafür erforderlichen Temperaturbereich erfolgt, so dass gleichzeitig mit der Laminierung auch die Sinterung ausgelöst wird.

Ein auf die Folienoberfläche 3u gerichteter Laserstrahl verdampft vorbestimmte Abschnitte der Folie 3 . Die verbleibenden Abschnitte der Folie 3 bilden Leiterbahnen 11 und einen elektrisch nicht angebundenen Folienkühlabschnitt 13, s. fig. 6.

Auf die in fig. 6 obere Seite werden weitere Schichten 9a und 9b auflaminiert, wie oben beschrieben, wobei die Schicht 9a ein thermoplastisches Polymer ist und die Schicht 9b ein Kunststoffverbundmaterial, vorliegend in einem Epoxyharz angeordnete Glasfasern, ist, s. fig. 7 . In diesen Schichten ist ein Kühlkanal 15 durch Bohren gebildet, der mit einem Kühlkörper 17 verbunden ist.

Die laminierten Schichten 9 , 9a , 9b und 19 mit den darin befindlichen, elektrischen Bauteilen 5 werden durch einen Laserstrahl in einzelne elektronische Baugruppen getrennt. In fig. 8 ist eine teilweise weggebrochene perspektivische Ansicht der auf diese Weise hergestellten Baugruppe 1 dargestellt, die nun für ihren Einsatz, bspw. in Mobiltelephonen, zur Verfügung steht.

Die Figur 9a zeigt in perspektivischer Ansicht die Folie 3 mit zwei elektronischen Bauteilen 5.1 und 5.2, die hier jeweils als sogenannte bare dies ausgebildet sind.

Die Figur 9b zeigt eine Draufsicht auf die Folie, die nicht unter die beanspruchte Erfindung fällt, wobei die Folie 3 mit den daran durch die Klebeverbindung fixierten Bauteilen 5.1 und 5.2. Zur Ausbildung der Klebeverbindung zwischen dem Bauteil 5.1 und der Folie 3 wird entlang der seitlichen Schnittkante 21 (vgl. auch Fig. 2), entlang derer das Bauteil 5.1 aus einem Wafer herausgeschnitten worden ist, der Kleber 20 aufgebracht, sodass die Klebeverbindung zwischen der seitlichen Kante 21 und der Oberfläche 30 der Folie 3 resultiert.

Dagegen wird der Kleber 20 zur Fixierung des Bauteils 5.2 auf der Folie 3 punktförmig im Bereich der Ecken des Bauteils 5.2 aufgebracht, wie ebenfalls in der Figur 9b dargestellt.

Die Figur 9c zeigt die entsprechenden Schnittansichten. Besonders vorteilhaft ist hier in beiden Fällen, dass die mögliche aktive Fläche für eine elektrische Kontaktierung der Bauelemente 5.1 bzw. 5.2 durch die Ausbildung der Klebeverbindung nicht bzw. nur unwesentlich verringert wird. Insbesondere steht trotz der Ausbildung der Klebeverbindung im Wesentlichen die gesamte Unterseite der Bauteile 5.1 bzw. 5.2, d.h. die Kontaktseite 5b, für eine elektrische Kontaktierung mit der Folie 3 zur Verfügung sowie auch die Oberseiten der Bauteile 5.1 und 5.2.

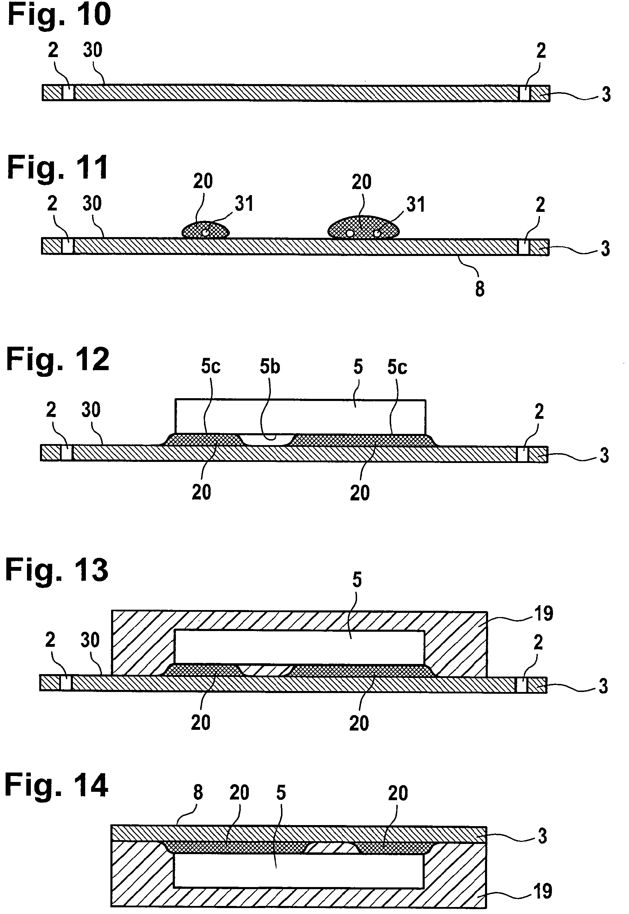

Fig. 10 bis fig. 14 zeigen schematisch einen Ausschnitt aus einer von einer Rolle abgewickelten leitfähigen Folie 3 gemäß einem weiteren Beispiel, das nicht unter die beanspruchte Erfindung fällt. Diese Folie 3 hat an bestimmten genau definierten Stellen Justagemarkierungen 2 , beispielsweise in Form von durch die Folie 3 hindurchgehende Löchern. Die Folie 3 ist auf beiden Seiten unbeschichtet. Es handelt sich beispielsweise um eine Kupferfolie.

Auf der Folie 3 wird auf der in den fig. 10 bis fig. 13 oberen Seite 30 an bestimmten Stellen, die in ihrer Position gegenüber den Justagemarkierungen 2 festgelegt sind, Kleber 20 aufgebracht. Das Aufbringen kann durch Drucken, Spritzen, Tropfen oder dergleichen geschehen. In dem Kleber können einzelne Abstandselemente 31, beispielsweise Glaskugeln einer bestimmten Größe, eingemischt sein. Mit der Größe der Spacer ist es möglich, verschiedene Dicken des Dielektrikums einzustellen. Der Kleber beinhaltet ferner Nanopartikel zur Ermöglichung des nachfolgenden Niedertemperatursinterns, vorzugsweise eines drucklosen Niedertemperatursinterns.

In einem weiteren Verfahrensschritt werden nun elektronische Bauteile mithilfe von geeigneten Einrichtungen auf die Stellen aufgesetzt, an denen vorher der Kleber 20 aufgebracht wurde, so dass die Kontaktstellen 5c der Bauteile mit dem Kleber 20 in Berührung kommen; dies ist in der fig. 12 exemplarisch für ein Bauteil 5 gezeigt, welches zwei Kontaktstellen 5c an dessen Kontaktseite 5b hat.

Diese elektronischen Bauteile 5 werden angedrückt, so dass die Kleberpunkte des Klebers 20 verquetscht werden und nur die Kontaktstellen 5c der elektronischen Bauteile 5 abdecken und gegenüber der Folie 3 verkleben. Dieser Zustand ist in fig. 12 dargestellt. Je nach Art des verwendeten Klebers können weitere Maßnahmen ergriffen werden, um das Aushärten des Klebers zu beschleunigen, beispielsweise eine zusätzliche oder ausschließliche UV-Härtung.

Anschließend kann in einem weiteren Verfahrensschritt, der nicht unbedingt erforderlich ist, um die elektronischen Bauteile 5 herum eine Umhüllung aus einem Füllmaterial 19, z.B. einer Polymermasse aufgebracht werden, die einzelne oder alle elektronischen Bauelemente in einer jeweils separaten oder einer gemeinsamen Umhüllung umhüllt. Durch diesen Vorgang kann die Sinterung der in dem Kleber 20 beinhalteten Nanopartikel ausgelöst werden, um die Verbindungen 23 (vgl. Fig. 4) herzustellen, wenn der Vorgang bei einer ausreichend hohen Temperatur durchgeführt wird.

Die Umhüllung reicht außerhalb der elektronischen Bauteile 5 bis zu der in den Fig. 10 bis fig. 13 oberen Seite 30 der Folie 3 . Auch zur Dimensionierung und Anordnung der Umhüllung aus der Polyestermasse gegenüber den elektronischen Bauelementen 5 können die Justagemarkierungen 2 dienen.

Da die Umhüllung Bauteile 5 umhüllt und bis zur Folie 3 reicht, gegebenenfalls auch mit der Folie 3 eine Art Verklebung eingeht, ist dadurch ein mechanisch stabiler Block gebildet. Vorzugsweise erstreckt sich die Umhüllung auch zwischen den Kontaktstellen, wie in der Fig. 13 und 14 gezeigt.

Anschließend an den Verfahrensschritt, dessen Ergebnis in fig. 13 dargestellt wurde, kann nun die elektronische Baugruppe zugeschnitten werden, das heißt aus der Folie 2 herausgeschnitten werden. Nun wird das Ergebnis des bisherigen Verfahrens umgedreht, so dass jetzt die freie Seite 8 der Folie 2 nach oben zu liegen kommt. Dies wird durch die Umhüllung und die damit verbundene Stabilisierung des in der Fig. 13 gezeigten Zwischenprodukts erleichtert, insbesondere der möglicherweise erforderliche Transport zwischen verschiedenen Fertigungsstationen.

In einem weiteren Schritt wird die mit der Umhüllung versehene Folie 3 mit einem Leiterplattenträger 9 laminiert, der auf der Seite der Folie 3 zu liegen kommt, auf der auch die elektronischen Bauteile 5 angeordnet sind (vgl. die Anordnung gemäß Fig. 5). Spätestens hierdurch wird die Sinterung und damit die Herstellung der Verbindungen 23 ausgelöst.

In einem weiteren Schritt wird nun die Folie 3 auf ihrer freien Seite 8 strukturiert, so dass jetzt Leiterbahnen erzeugt sind.

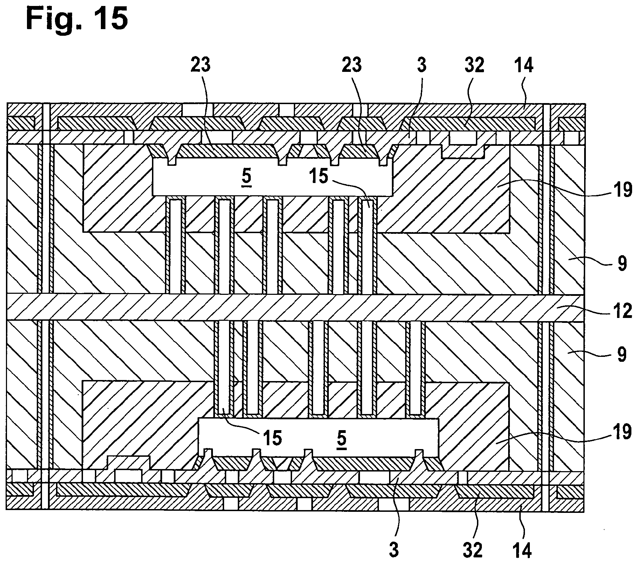

Diese elektronische Baugruppe kann aber weiterverarbeitet und weiter ausgestaltet werden. fig. 15 zeigt eine solche weitere Ausgestaltung einer elektronischen Baugruppe, wobei praktisch zwei Baugruppen, wie in fig. 14 dargestellt, Rücken an Rücken miteinander verbunden werden unter Zwischenlage einer metallischen Schicht 12 , die zwischen den beiden Leiterplattenträgern 9 angeordnet ist. Diese metallische Schicht 12 dient beispielsweise zur Wärmeabfuhr der in den elektronischen Bauteilen 5 erzeugten Wärme.

Die beiden hier zusammengefassten Baugruppen unterscheiden sich von der einfachen Baugruppe der fig. 14 noch darin, dass auf die in fig. 14 noch obere metallische Schicht, d.h. die Folie 3, unter Zwischenlage einer Isolierungsschicht 32 eine weitere metallische leitende Schicht 14 aufgebracht ist, die durch Öffnungen in der Isolierungsschicht 32 wiederum Kontakte mit Leiterbahnen der ursprünglich oberen leitenden Schicht 2 herstellt.

Von der nicht aktiven Seite der elektronischen Bauteile 5 führen metallisierte Kühlkanäle 15 durch den Leiterplattenträger 9 hindurch zu der mittleren Kühlschicht 12, so dass die Wärme hier abgeleitet werden kann.

In fig. 15 ist als Beispiel, das nicht unter die beanspruchte Erfindung fällt, gezeigt, dass die obere der beiden Lagen elektronische Bauteile 5 ohne eine Umhüllung aufweist, während die untere umgedrehte Anordnung eine Umhüllung enthält.

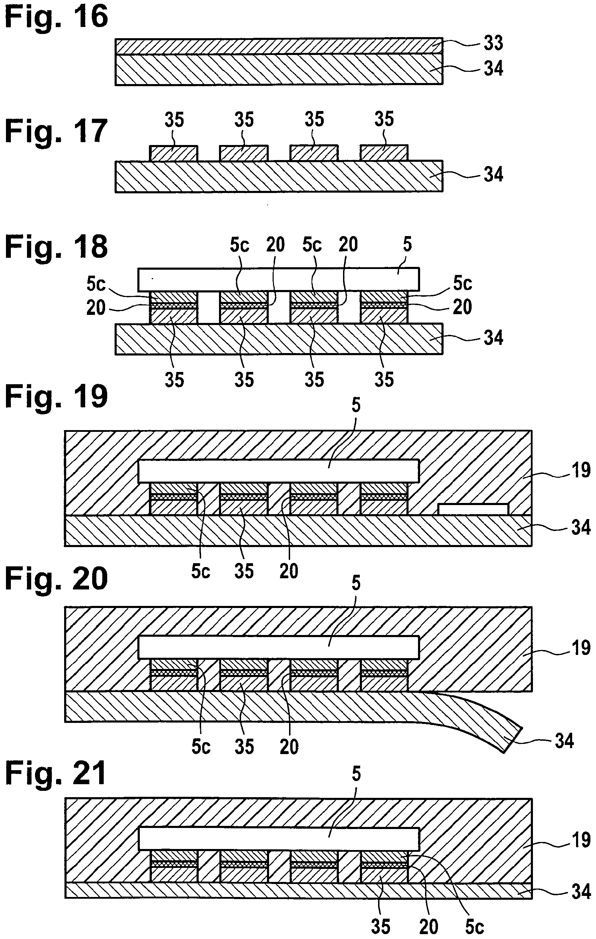

In den fig. 16 bis fig. 21 wird ein Beispiel, das nicht unter die beanspruchte Erfindung fällt dargestellt. Die fig. 16 bis fig. 21 zeigen Zustände bei der Durchführung des Verfahrens, die etwa den fig. 10 bis fig. 14 entsprechen.

Während in fig. 10 eine beidseits unbeschichtete Folie 3 als Ausgangsmaterial verwendet wird, verwendet die in den fig. 16 bis fig. 21 dargestellte Ausführungsform als Ausgangsmaterial eine Folie 33 , die an einer Trägerfolie 34 angebracht ist. Die Trägerfolie 34 kann aus Metall, aus Keramik oder auch aus Polymer bestehen. Die leitende Folie 33 besteht beispielsweise aus Kupfer und ist nicht mit Nanopartikeln beschichtet.

In einem ersten Verfahrensschritt wird die leitende Folie 33 in der Weise strukturiert, dass Anschlusspads 35 auf der Trägerfolie 34 ausgebildet werden, die den Anschlussstellen 5c der zu befestigenden elektronischen Bauteile entsprechen.

Ein solches elektronisches Bauteil 5 ist in fig. 18 dargestellt. Dieses elektronische Bauteil 5 wird nun mit seinen Anschlussstellen 5c mit den Anschlusspads 35 verbunden. Dies kann beispielsweise dadurch geschehen, dass auf die Anschlusspads 35 oder die diesen zugewandten Seiten der Anschlussstellen 5c des elektronischen Bauteils 5 Kleber 20 aufgebracht wird.

Bei dem Kleber 20 handelt es sich um einen Kleber mit in dem Kleber verteilten Nanopartikeln. Dadurch wird eine Klebschicht gebildet, die jeweils zwischen den Anschlusspads 35 und den Anschlussstellen 5c des elektronischen Bauelements 5 vorhanden ist. Auf diese Weise wird das elektronische Bauteil 5 mit seinen Anschlussstellen 5c den Anschlußpads 35 zugewandt mit diesen verbunden, nämlich zunächst nur über eine Klebeverbindung.

Anschließend kann das elektronische Bauteil 5 mit einer Umhüllung aus einem Füllmaterial 19 , wiederum aus Polymermasse, umgeben werden, die das gesamte elektronische Bauteil 5 einschließlich der Anschlusspads 35 umgibt und bis zu der Trägerfolie 34 reicht. Das Ergebnis ist in fig. 19 dargestellt.

Anschließend kann die Trägerfolie 34 abgetrennt werden, was in fig. 20 gezeigt ist. Die weitere Behandlung der Umhüllung oder einer Laminierung und des darin enthaltenen elektronischen Bauelements 5 geschieht in der bei Leiterplatten üblichen Weise. Wesentlich ist, dass bei der Umhüllung mit dem Füllmaterial oder der Laminierung ein Temperaturbereich erreicht wird, im dem die Niedertemperatursinterung der in dem Kleber 20 beinhalteten Nanopartikel ausgelöst wird, um die Verbindungen 23 (vgl. Fig. 4) herzustellen.
fig. 20 zeigt das Ablösen der Trägerschicht 34 . Anstelle des Ablösens der Trägerschicht 34 ist es auch möglich, die Trägerschicht 34 nicht abzulösen, sondern weiter zu strukturieren, beispielsweise durch Ätzen, durch Abtragen oder dergleichen. Dies ist in fig. 21 dargestellt.

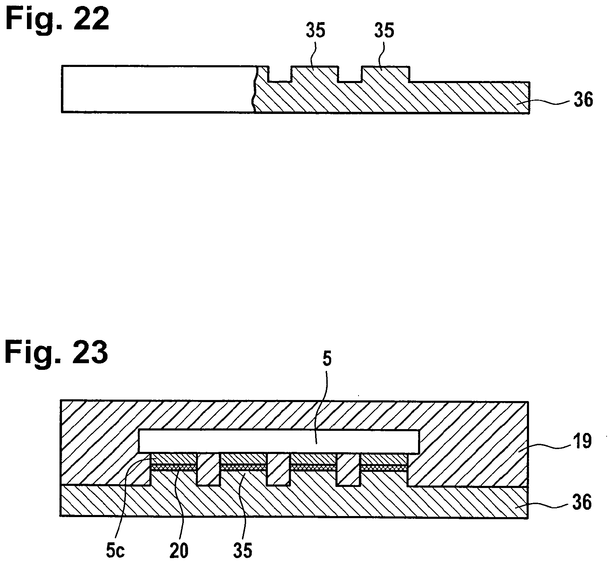

Die fig. 22 und fig. 23 zeigen ein weiteres Beispiel, das nicht unter die beanspruchte Erfindung fällt, wobei als Ausgangsfolie eine dickere Folie 36 verwendet wird, die dann ähnlich wie in fig. 17 auf ihrer den elektronischen Bauteilen zugewandten Seite zunächst strukturiert wird, um dadurch Anschlusspads 35 zu bilden. Links in fig. 22 ist der Ausgangszustand dieser dickeren Folie 36 dargestellt, während rechts in fig. 22 das Ergebnis der Strukturierung dargestellt ist.

Auf die so hergestellte strukturierte Folie wird dann das elektronische Bauteil 5 in der gleichen Weise aufgesetzt, mit Hilfe des Kleber 20 befestigt, wie dies in fig. 18 dargestellt wurde. Auch hier kann nach dem Befestigen des elektronischen Bauteils 5 und der Umhüllung eine Strukturierung erfolgen.

Die elektronischen Baugruppen, wie sie in fig. 20, fig. 21 und fig. 23 dargestellt sind, können dann in der gleichen Weise zu Mehrlagenbaugruppen zusammengefasst werden, wie dies bei der ersten Ausführungsform beschrieben wurde.

Das von der Erfindung vorgeschlagene Verfahren ermöglicht es, auf elektronischen Schaltungsträgern eine erheblich gesteigerte Flächennutzung zu verwirklichen. Es können zusätzliche Lagen mit auf engstem Raum bestückten Bauelementen hergestellt werden, sowohl aktiven als auch passiven Bauelementen. Die passiven und aktiven elektronischen Bauelemente können kostengünstig verkapselt werden, wodurch eine hohe Zuverlässigkeit erreicht wird. Es werden risikoreiche Mischtechniken, nämlich Löten, Kleben und Drahtbonden in der Fertigung vermieden. Durch eine planare Ausgangsstruktur lassen sich reproduzierbare HF-Übergänge verwirklichen. Von besonderem Vorteil ist, dass kein gesonderter Prozessschritt für die Ausbildung der elektrischen Verbindungen erforderlich ist, da dieses durch Niedertemperatursinterung zusammen mit der Umhüllung oder der Laminierung erfolgt, und dass hierdurch gleichzeitig eine besonders stabile mechanische Verbindung und eine elektrische Verbindung mit einem geringen Übergangswiderstand sowie einer geringen Länge resultiert, was für Hochfrequenzanwendungen vorteilhaft ist.

### Bezugszeichenliste

- 1:: elektronische Baugruppe
- 2:: Justagemarkierung
- 3:: elektrisch leitende Folie
- 3a:: Trägerfolie
- 3o:: obere Seite der elektrisch leitenden Folie
- 3u:: untere Seite der elektrisch leitenden Folie
- 5:: elektrisches Bauteil
- 5a:: Schutzschicht
- 5b:: Kontaktseite
- 5c:: elektrische Kontaktstelle
- 8:: freie Seite
- 9:: Träger
- 9a:: Trägerschicht
- 9b:: Verstärkungsschicht
- 11:: Leiterbahnen
- 12:: metallische Schicht
- 13:: Folienkühlabschnitt
- 14:: Schicht
- 15:: Kühlkanal
- 17:: Kühlkörper
- 19:: Füllmaterial
- 20:: Kleber
- 21:: Kante
- 22:: Oberseite
- 23:: Verbindung
- 30:: obere Seite
- 31:: Abstandselement
- 32:: Isolierungsschicht
- 33:: Folie
- 34:: Trägerfolie
- 35:: Anschlusspads
- 36:: Folie
- 37:: Nanopartikel

## Patentansprüche

1. Verfahren zum Herstellen einer elektronischen Baugruppe (1)mit den Schritten:
1.1 Bereitstellen einer elektrisch leitenden Folie (3), insbesondere einer Trägerfolie (3a),
1.2 Bereitstellen mindestens eines elektrischen Bauteils (5) mit mindestens einer elektrischen Kontaktstelle (5c),
1.3 Aufbringung eines Klebers (20) zwischen dem elektrischen Bauteil und einer Oberfläche (30) der elektrisch leitenden Folie,
1.4 Anordnen des mindestens einen Bauteils (5) mit der mindestens einen elektrischen Kontaktstelle (5c) auf der Oberfläche (30) der elektrisch leitenden Folie (3), und Fixierung des mindestens einen Bauteils durch Ausbildung einer Klebeverbindung zwischen dem elektrischen Bauteil und der Oberfläche,
1.5 Bereitstellen eines Trägers (9), insbesondere aus einem flexiblen Material,
1.6 Laminieren der Folie (3) mit dem Träger (9) derart, dass das mindestens eine elektrische Bauteil (5) zwischen Folie (3) und Träger (9) angeordnet ist, und Bilden einer mechanischen und elektrischen Verbindung (23) zwischen der elektrischen Kontaktstelle des mindestens einen elektrischen Bauteils (5) und der elektrisch leitenden Folie (3) durch Niedertemperatursintern von Nanopartikeln,
1.7 Strukturieren der elektrisch leitenden Folie (3) zu Leiterbahnen (11) und/oder Kühlflächen (13), **dadurch gekennzeichnet, dass** das Laminieren der Folie gleichzeitig mit dem Niedertemperatursintern erfolgt.

2. Verfahren nach Anspruch 1, wobei das Laminieren und das Niedertemperatursintern bei 130-300 Grad Celsius oder zwischen 150 - 250 Grad Celsius oder bei 170 bis 190 Grad Celsius oder bei 180 Grad Celsius erfolgt.

3. Verfahren nach Anspruch 1 oder 2, wobei der Kleber die Nanopartikel beinhaltet, und der Kleber auf die mindestens eine elektrische Kontaktstelle aufgebracht wird.

4. Verfahren nach Anspruch 3, wobei durch das Niedertemperatursintern gesinterte Nanopartikel ausgebildet werden, die in einer von dem Kleber gebildeten Matrix eingebettet sind.

5. Verfahren nach einem der vorhergehenden Ansprüche, wobei das Niedertemperatursintern bei einem Druck von 15 - 20 bar, d.h. 1500- 2000 kPa, durchgeführt wird.

6. Verfahren nach einem der vorhergehenden Ansprüche, wobei das Laminieren der Folie (3) mit dem Träger (9) im Schritt 1.6 durch Ausüben eines Anpressdruckes auf die Folie (3) in Richtung des Trägers (9) unter gleichzeitiger Wärmeeinwirkung erfolgt.

7. Verfahren nach einem der vorhergehenden Ansprüche, **gekennzeichnet durch** ein Umhüllen des mindestens einen elektrischen Bauteils nach dem Schritt 1.4 mit einem Füllmaterial (19) aus einem Polymer.

8. Verfahren nach Anspruch 8, **dadurch gekennzeichnet, dass** der Polymer ein Thermoplast, ein Duroplast, ein Elastomer oder ein Liquid Crystal Polymer ist.

9. Verfahren nach einem der vorhergehenden Ansprüche, **gekennzeichnet dadurch, dass** nach dem Schritt 1.7 ein Aufbringen mindestens einer Verstärkungsschicht (9b) auf den Träger (9) oder die Trägerschicht (9a) erfolgt, oder dass der Träger (9) nach dem Schritt 1.6 entfernt wird.

10. Verfahren nach einem der vorhergehenden Ansprüche, **gekennzeichnet durch** ein Zuschneiden des Trägers (9) in eine vorbestimmte Form zum Bilden der elektronischen Baugruppe (1) vor oder nach dem Laminieren mit dem Träger (9) im Schritt 1.6.

11. Verfahren nach einem der vorhergehenden Ansprüche, wobei es sich bei den Nanopartikeln um Silber einer Partikelgröße zwischen 20 - 300nm handelt.

12. Verfahren nach einem der vorhergehenden Ansprüche, wobei die Oberfläche der Folie mit einer gleichmäßigen Beschichtung von 200-300nm Silber oder einer silberhaltigen Legierung versehen ist.

13. Verfahren nach einem der vorhergehenden Ansprüche, wobei die Nanopartikel aus Gold, Silber, Nickel oder Kupfer oder aus einer Legierung aus diesen Metallen gebildet sind.

## Claims

1. A method for producing an electronic assembly (1), comprising the following steps:
1.1 providing an electrically conducting film (3), in particular a carrier film (3a);
1.2 providing at least one electrical component (5) including at least one electrical contact site (5c);
1.3 applying an adhesive (20) between the electrical component and a surface (30) of the electrically conducting film;
1.4 arranging the at least one component (5) including the at least one electrical contact site (5c) on the surface (30) of the electrically conducting film (3), and fixing the at least one component by forming an adhesive joint between the electrical component and the surface;
1.5 providing a carrier (9), in particular made of a flexible material;
1.6 laminating the film (3) with the carrier (9) in such a way that the at least one electrical component (5) is arranged between the film (3) and the carrier (9), and forming a mechanical and electrical connection (23) between the electrical contact site of the at least one electrical component (5) and the electrically conducting film (3) by low-temperature sintering of nanoparticles;
1.7 structuring the electrically conducting film (3) to form conductor tracks (11) and/or cooling surfaces (13), **characterized in that** the lamination of the film takes place simultaneously with the low-temperature sintering.

2. The method according to claim 1, wherein the lamination and the low-temperature sintering take place at 130 to 300 degrees Celsius, or between 150 and 250 degrees Celsius, or at 170 to 190 degrees Celsius, or at 180 degrees Celsius.

3. The method according to claim 1 or 2, wherein the adhesive includes the nanoparticles, and the adhesive is applied to the at least one the electrical contact site.

4. The method according to claim 3, wherein nanoparticles sintered by the low-temperature sintering are formed, which are embedded in a matrix formed by the adhesive.

5. The method according to any one of the preceding claims, wherein the low-temperature sintering is carried out at a pressure of 15 to 20 bar, that is 1500 to 2000 kPa.

6. The method according to any one of the preceding claims, wherein the lamination of the film (3) with the carrier (9) in step 1.6 is carried out by exerting a pressing pressure on the film (3) in the direction of the carrier (9), while applying heat at the same time.

7. The method according to any one of the preceding claims, **characterized by** enveloping the at least one electrical component after step 1.4 with a filler material (19) made of a polymer.

8. The method according to claim 8, **characterized in that** the polymer is a thermoplastic material, a thermoset material, an elastomer, or a liquid crystal polymer.

9. The method according to any one of the preceding claims, **characterized in that**, after step 1.7, at least one reinforcement layer (9b) is applied to the carrier (9) or the carrier layer (9a), or that the carrier (9) is removed after step 1.6.

10. The method according to any one of the preceding claims, **characterized by** cutting the carrier (9) into a predetermined shape for forming the electronic assembly (1), prior to and/or after lamination with the carrier (9) in step 1.6.

11. The method according to any one of the preceding claims, wherein the nanoparticles are silver having a particle size between 20 and 300 nm.

12. The method according to any one of the preceding claims, wherein the surface of the film is provided with a uniform coating of 200 to 300 nm silver or a silvercontaining alloy.

13. The method according to any one of the preceding claims, wherein the nanoparticles are formed of gold, silver, nickel or copper or of an alloy made of these metals.

## Revendications

1. Procédé de fabrication d'un module électronique (1) avec les étapes :
1.1 fourniture d'une feuille (3) électriquement conductrice, notamment une feuille support (3a),
1.2 fourniture d'au moins un composant (5) électrique avec au moins un point de contact électrique (5c),
1.3 dépôt d'un adhésif (20) entre le composant électrique et une surface (30) de la feuille électriquement conductrice,
1.4 agencement de l'au moins un composant (5) avec l'au moins un point de contact électrique (5c) sur la surface (30) de la feuille (3) électriquement conductrice, et fixation de l'au moins un composant par la création d'une liaison adhésive entre le composant électrique et la surface,
1.5 fourniture d'un support (9), notamment à base d'un matériau souple,
1.6 laminage de la feuille (3) avec le support (9) de telle façon que l'au moins un composant (5) électrique soit disposé entre la feuille (3) et le support (9), et formation d'une liaison (23) mécanique et électrique entre le point de contact électrique de l'au moins un composant (5) électrique et la feuille (3) conductrice électriquement par un frittage à basse température de nanoparticules,
1.7 structuration de la feuille (3) électriquement conductrice en pistes conductrices (11) et/ou en surfaces de refroidissement (13), **caractérisé en ce que** le laminage de la feuille a lieu simultanément au frittage à basse température.

2. Procédé selon la revendication 1, dans lequel le laminage et le frittage à basse température ont lieu à 130 - 300 degrés Celsius, ou entre 150 et 250 degrés Celsius, ou à 170 à 190 degrés Celsius, ou à 180 degrés Celsius.

3. Procédé selon la revendication 1 ou la revendication 2, dans lequel l'adhésif contient des nanoparticules, et l'adhésif est rapporté sur l'au moins un point de contact électrique.

4. Procédé selon la revendication 3, dans lequel des nanoparticules frittées, qui sont incorporées dans une matrice formée par l'adhésif, sont créées par le frittage à basse température.

5. Procédé selon l'une des revendications précédentes, dans lequel le frittage à basse température est exécuté à une pression de 15 à 20 bar, c'est-à-dire de 1500 à 2000 kPa.

6. Procédé selon l'une des revendications précédentes, dans lequel le laminage de la feuille (3) avec le support (9) dans l'étape 1.6 a lieu par l'exercice d'une pression d'appui sur la feuille (3) en direction du support (9) sous l'influence simultanée de chaleur.

7. Procédé selon l'une des revendications précédentes, **caractérisé par** un enveloppement de l'au moins un composant électrique après l'étape 1.4 avec un matériau de remplissage (19) à base d'un polymère.

8. Procédé selon la revendication 8, **caractérisé en ce que** le polymère est un thermoplastique, un duroplastique, un élastomère ou un polymère cristal liquide.

9. Procédé selon l'une des revendications précédentes, **caractérisé en ce qu'**après l'étape 1.7, il y a une application d'au moins une couche de renforcement (9b) sur le support (9) ou la couche support (9a), ou que le support (9) est retiré après l'étape 1.6.

10. Procédé selon l'une des revendications précédentes, **caractérisé par** un découpage du support (9) dans une forme prédéterminée pour former le module électrique (1) avant ou après le laminage avec le support (9) dans l'étape 1.6.

11. Procédé selon l'une des revendications précédentes, dans lequel, dans le cas des nanoparticules, il s'agit d'argent avec une taille particulaire entre 20 et 300 nm.

12. Procédé selon l'une des revendications précédentes, dans lequel la surface de la feuille est munie d'un revêtement régulier de 200 à 300 nm d'argent ou d'un alliage contenant de l'argent.

13. Procédé selon l'une des revendications précédentes, dans lequel les nanoparticules sont formées à base d'or, d'argent, de nickel ou de cuivre ou d'un alliage à base de ces métaux.
